# EUROPÄISCHE PATENTSCHRIFT

(11) **EP 2 190 375 B1**
(45) Veröffentlichungstag und Bekanntmachung des Hinweises auf die Patenterteilung: **04.06.2014**
(21) Anmeldenummer: 08785435.2
(22) Anmeldetag: 07.08.2008
(51) Int. Cl.: A61B 19/00, A61B 19/02

(54) **OPERATIONSHILFSVORRICHTUNG**
OPERATING AID
DISPOSITIF D'ASSISTANCE OPÉRATOIRE

(30) Priorität: 08.08.2007 DE 202007011044 U
(43) Veröffentlichungstag der Anmeldung: 02.06.2010
(73) Patentinhaber: Podda-Heubach, Silvio, New York, NY 10010 (US)
(72) Erfinder: Podda-Heubach, Silvio, New York, NY 10010 (US)
(74) Vertreter: Hagemann, Heinrich
(86) Internationale Anmeldenummer: PCT/EP2008/006529
(87) Internationale Veröffentlichungsnummer: WO 2009/019021

(56) Entgegenhaltungen:
- WO-A-2004/080236
- GB-A- 191 030 395
- JP-A- 2003 219 904
- US-A- 2 176 052
- US-A- 2 824 681
- US-A- 5 353 974

## Beschreibung

Die vorliegende Erfindung betrifft einen Halter zum Bereitstellen von Operationsinstrumenten für den unmittelbaren Zugriff eines Operateurs während einer Operation am menschlichen oder tierischen Körper.

Ein operativer Eingriff am menschlichen oder tierischen Körper erfordert die wiederholte Ausführung bestimmter Tätigkeiten, Handgriffe und Bewegungen. Insbesondere erfordert ein operativer Eingriff den ständigen Austausch von Operationsinstrumenten zwischen dem Operateur und einer assistierenden Person. In bestimmten Phasen eines Eingriffs, in denen mehrere verschiedene Instrumente im schnellen Wechsel benötigt werden, bedingt dieser Austausch einen unnötigen zusätzlichen Zeitaufwand und birgt zudem ein erhöhtes Verletzungsrisiko für alle Beteiligten mit sich, und zwar unabhängig davon, ob der operative Eingriff in einer Klinik, im Rahmen einer ambulanten Behandlung, an einem Unfallort oder in einer ärztlichen Praxis vorgenommen wird. Der ständige Austausch von Operationsinstrumenten zwischen Operateur und Assistenzpersonal ist unter den herkömmlichen Gegebenheiten jedenfalls unvermeidbar und hat mögliche Risiken und Folgen.

Auch kommt es nicht selten vor, dass ein benötigtes Instrument nicht schnell genug aufgefunden werden kann, aus dem Operationsbereich herausfällt und kontaminiert wird oder, schlimmer noch, während der Operation verloren geht. Auch längere Verzögerungen bei der Bereitstellung der Operationsinstrumente sind im klinischen Alltag nicht ungeöhnlich und treten insbesondere dann auf, wenn während längerer Operationen mehrfach das Assistenzpersonal wechselt. Ein erhöhtes Verletzungsrisiko für Patient und Operateur besteht insbesondere auch beim Nähen längerer Schnitte, wenn Scheren, Pinzetten und Nadelhalter üblicherweise auf einem stationären Ständer ("Mayo stand") im Operationsbereich oder auf einem Tablett, direkt auf dem Patienten, bereitgehalten werden.

Es ist daher erstrebenswert, die ergonomischen Bedingungen während eines operativen Eingriffs in einer Weise zu verbessern, die dem Operateur den unmittelbaren Zugriff auf eine bestimmte Anzahl von Instrumenten während der Operation ermöglicht und die den zusätzlichen Zeitaufwand durch das ständige Bewegen der benötigten Instrumente in den Operationsbereich hinein und aus diesem wieder heraus reduziert sowie das Verletzungsrisiko für alle Beteiligten mindert.

Aus dem Stand der Technik sind ähnliche Problemstellungen bei der Verrichtung bestimmter handwerklicher Tätigkeiten bekannt, bei denen im häufigen Wechsel Werkzeuge zur Erfüllung bestimmter Aufgaben unmittelbar vor Ort benötigt werden. So ist etwa im Schneiderhandwerk ein unmittelbarer Zugriff auf Nadeln während der Anprobe eines Kleidungsstückes erforderlich. Zur Bereitstellung der benötigten Nadeln wird hierbei in der Regel ein sogenanntes Nadelkissen verwendet. Dabei handelt es sich um ein kleines, beispielsweise halbkugelförmiges Kissen, in welches die Nadeln zur Entnahme gesteckt sind und das eine feste Verbindung mit einem Armhalter aufweist, der im Gebrauch vom Benutzer am Unterarm getragen wird. Derartige Nadelkissen ermöglichen eine unmittelbare Bereitstellung der benötigten Nadeln auf ergonomische Weise und erlauben ein zügiges und zeitsparendes Arbeiten.

Allerdings ist ein solches Nadelkissen ersichtlich nur für die Bereitstellung sehr kleiner und insbesondere leichter Werkzeuge, die zudem in das Nadelkissen steckbar sein müssen, geeignet. Jeder Versuch, damit ein schwereres Instrument zu haltern, muss allein schon wegen der elastischen Eigenschaften des Kissenmaterials fehlschlagen. Mit anderen Worten: eine derartige Verwendung ist allein schon wegen der damit verbundenen Verletzungsgefahr für den Anwender und/oder der Gefahr einer Beschädigung des Instruments beim Herunterfallen ausgeschlossen.

Die Aufgabe der Erfindung besteht darin, auch vergleichsweise große und schwere magnetische Operationsinstrumente unterschiedlichster Form auf ergonomische und sichere Weise während eines operativen Eingriffs unmittelbar bereitzustellen und insoweit zu einer Zeitersparnis bei der Operation, wie einer weitgehenden Reduzierung des Verletzungsrisikos für allen Beteiligten beizutragen. Diese Aufgabe wird dadurch gelöst, dass bei einem Halter mit einem Haltemittel zum Fixieren der Instrumente, einem Befestigungsmittel zur lösbaren Befestigung des Haltemittels am Arm eines Benutzers und einem Verbindungsmittel zum Herstellen einer Verbindung zwischen dem Befestigungsmittel und dem Haltemittel gemäß dem Oberbegriff des Anspruchs 1, das Verbindungsmittel im Wesentlichen zwei planparallele Scheiben umfasst, die um eine gemeinsame orthogonale Mittelachse drehbar gelagert und durch mechanische oder magnetische Mittel in ihrer planparallelen Zuordnung gehalten sind, wobei eine der Scheiben ein Unterteil des Verbindungsmittels bildet, die mit dem Befestigungsmittel drehfest verbunden ist und die Grundplatte darstellt, und die andere Scheibe ein Oberteil des Verbindungsmittels bildet, das mit dem Haltemittel verbunden ist.

Das Dokument US 5353974 offenbart alle Merkmale des Oberbegriffs des Anspruchs 1.

Der erfindungsgemäße Halter ermöglicht in vorteilhafter Weise die ergonomische Bereitstellung ausgewählter Instrumente in jede Phase einer Operation. Insbesondere ermöglicht der erfindungsgemäße Halter, dass der Operateur während eines operativen Eingriffs jederzeit und unmittelbar Zugriff auf die jeweils erforderlichen Instrumente mit der dominanten Hand hat, ohne dazu auf die Hilfe von Assistenzpersonal und/oder von stationären Haltevorrichtungen bekannter Art angewiesen zu sein.

Als besonders vorteilhaft erweist sich der erfindungsgemäße Halter dadurch, dass er dem Operateur die alleinige Kontrolle über die unmittelbar benötigten Instrumente ermöglicht und insoweit unabhängig von der Aufmerksamkeit des Assistenzpersonals macht.

Ein weiterer Vorteil besteht darin, dass der erfindungsgemäße Halter zur Optimierung der Operationsbedingungen beiträgt, indem er einen ergonomischen Zugriff auf die wesentlichen Operationsinstrumente während eines Eingriffs ermöglicht. Mit anderen Worten: Durch Verwendung des erfindungsgemäßen Halters reduzieren sich Bewegungsaufwand und Raumbedarf des Operateurs während eines Eingriffs in vorteilhafter Weise, was wiederum zu einer geringeren Ermüdung des Operateurs beiträgt und auch das Ablenkungsrisiko für den Operateur reduziert, was wiederum die Sicherheit für alle Beteiligten erhöht. Somit vermag die Erfindung auch die Produktivität und die Arbeitsqualität des Operateurs zu erhöhen.

Weiterhin ist von Vorteil, dass sich durch die Verwendung des erfindungsgemäßen Halters die Gefahr einer Kontamination der Operationsinstrumente während eines Eingriffs deutlich reduzieren lässt, indem der Zugriff auf die Instrumente unmittelbar durch den Operateur selbst erfolgt. Einhergehend damit reduziert sich das Verletzungsrisiko für das Assistenzpersonal, den Operateur und den Patienten.

In Fällen, in denen beide Unterarme des Operateurs frei sein müssen, wie beispielsweise bei tiefen Eingriffen, kann der Halter ohne Weiteres auf den Oberarm verschoben werden und dort gegebenenfalls zusätzlich gesichert werden. Natürlich ist es auch möglich, den Halter in einem solchen Fall ganz abzunehmen und in einer sterilen Umgebung bis zu einer Weiterverwendung vorzuhalten.

Durch den einfachen Aufbau des Halters ist eine problemlose und schnelle Anpassung an die spezifischen Erfordernisse unterschiedlicher medizinischer Einsatzfälle und Gebiete jederzeit möglich, indem etwa das Haltemittel bzw. Tablett entsprechend ausgebildet wird und/oder spezielle Adapter verwendet werden.

Von besonderem Vorteil erweist sich auch die kleine Baugröße und das sehr geringe Gewicht des Halters auf dem Unterarm des Operateurs.

Schließlich ergibt sich ein weiterer Vorteil aus der Tatsache, dass der erfindungsgemäße Halter nur aus wenigen, robusten, leicht zu reinigenden und zu sterilisierenden Teilen besteht, die zudem einfach aufgebaut sind und kostengünstig mit den konventionellen Fertigungsmethoden der Metall- und Kunststofftechnik, unter Verwendung von auf dem Gebiet medizinischer Geräte üblicher Materialien, herstellbar sind.

Vorteilhafte Ausführungsformen der Erfindung ergeben sich aus den Unteransprüchen und werden im Folgenden weitergehend erläutert:

Der erfindungsgemäße Halter, der auch als persönliche Operationshilfsvorrichtung ("Personal Assistant Surgical Device" - PASD) bezeichnet werden kann, weist das Haltemittel in Form einer magnetischen Plattform bzw. eines magnetischen Tabletts zur lösbaren Ablage der benötigten Operationsinstrumente auf, welches auf der Oberseite des nichtdominanten Vorderarms des Operateurs lösbar anbringbar ist. Form, Größe und Gewicht des Tabletts richten sich dabei nach der Art der durchzuführenden Operation und der Größe bzw. Anzahl der zu halternden Instrumente und sind somit in weiten Grenzen entsprechend festzulegen. Bevorzugt weist das Tablett eine rechteckige, quadratische, ovale oder runde Form auf und ermöglicht eine Ablage der Instrumente in im Wesentlichen paralleler Anordnung.

Nach einer ersten Ausführungsform ist die Oberseite des Haltemittels eben ausgebildet. Weitere Ausführungsformen des Haltemittels weisen auf der Ablagefläche spezifische Rillen, Vertiefungen und/oder Erhebungen zur zweckmäßigen Ausrichtung und Verbesserung der Lagestabilität einzelner, häufig benötigter Instrumente, wie etwa Pinzetten, Scheren und Skalpelle - ohne allerdings darauf beschränkt zu sein -, auf. Vorzugsweise ermöglichen die spezifischen Rillen, Vertiefungen und/oder Erhebungen eine parallele Ausrichtung der Instrumente.

Die Mittel zum Erzeugen eines magnetischen Feldes sind vorzugsweise so angeordnet und dimensioniert, dass sich die Feldwirkung im Wesentlichen über die gesamte Oberseite des Haltemittels bzw. Tabletts erstreckt, was eine Halterung der abgelegten Operationsinstrumente in beliebiger Orientierung ermöglicht. Eine ganzflächige Feldverteilung eignet sich insbesondere zum Haltern relativ kleiner Instrumente. Weitere Ausführungsformen des Haltemittels weisen die magnetische Kraftwirkung insbesondere punktrasterförmig, zum Haltern größerflächiger Instrumente, oder in Streifen von im Wesentlichen paralleler Ausrichtung, zum Haltern von dazu orthogonal angeordneten Instrumenten sowohl auf der Oberseite als auch auf der Unterseite des Haltemittels, auf.

Die Oberseite des Haltemittels ist im Wesentlichen parallel zur Unterseite ausgebildet. Nach einer weiteren Ausführungsform weist das Tablett zum besonders ergonomischen Zugriff auf die Instrumente während der Operation eine gegenüber der Unterseite geneigte Oberseite auf, wobei Neigungswinkel zwischen 0 und 30 Grad, insbesondere zwischen 5 und 15 Grad, bevorzugt sind.

Um eine für den Operateur ergonomisch jeweils optimale Ausrichtung des Haltemittels bzw. Tabletts auf dem Unterarm während der Operation zu gewährleisten, ist dieses schwenkbar oder drehbar mit einer festen Grundplatte verbunden. Durch die angegebenen Arretierungsmittel kann das Tablett in jeder beliebigen Winkelposition relativ zu Grundplatte gehalten werden. Der Halter ist auf diese Weise gleichermaßen vorteilhaft sowohl auf dem linken wie auf dem rechten Arm eines Operateurs verwendbar, bzw. eignet sich gleichermaßen für Rechtshänder wie für Linkshänder.

Die relative Positionierung des Haltemittels zum Befestigungsmittel und somit zum Arm des Benutzers wird durch das Verbindungsmittel ermöglicht. Das Verbindungsmittel umfasst dazu im Wesentlichen zwei planparallele Scheiben, die um eine gemeinsame orthogonale Mittelachse drehbar gelagert sind und die durch mechanische oder magnetische Mittel in ihrer planparallelen Zuordnung gehalten werden. Die Relativdrehung bzw. Winkelzuordnung zwischen den beiden planparallelen Scheiben kann dabei sowohl kontinuierlich als auch schrittweise erfolgen. Im Gebrauchszustand des Halters weist eine der beiden Scheiben - das Unterteil des Verbindungsmittels - eine drehfeste Verbindung mit dem Befestigungsmittel auf, während die andere Scheibe - das Oberteil des Verbindungsmittels - mit dem Haltemittel verbunden ist.

Die magnetische Verbindung zwischen dem Oberteil des Verbindungsmittels und dem Haltemittel, genauer zwischen der Oberseite des Oberteils und der Unterseite des Haltemittels bzw. Tabletts wird insoweit ermöglicht, als das Oberteil, jedenfalls teilweise, aus einem magnetischen Material gefertigt ist, das durch das magnetische Felderzeugungsmittel des Halteteils angezogen wird. Die Haltekraft hängt ab von der Größe des wirksamen Verbindungsbereichs, der magnetischen Feldstärke im Verbindungsbereich sowie von den magnetischen Eigenschaften des für das Oberteil verwendeten Materials und ist insoweit eindeutig festlegbar. Maßgebend für die Auslegung der magnetisch wirksamen Parameter ist die zu halternde Gesamtmasse, nämlich die Masse des Oberteils, des Haltemittels und der zu halternden Operationsinstrumente. Um eine definierte Winkelbeziehung zwischen Oberteil und Haltemittel im Gebrauchszustand des Halters sicherzustellen, kann das Oberteil in Form einer Aufnahme mit Seitenwänden ausgebildet sein, die das Haltemittel lateral begrenzen. Vorzugsweise überragen diese Seitenwände das Haltemittel, wodurch eine mechanische Barriere geschaffen wird, die ein Abgleiten der auf der Oberseite des Haltemittels abgelegten Instrumente verhindert.

Zur drehfesten Verbindung des Unterteils des Verbindungsmittels mit dem Befestigungsmittel, genauer der Unterseite des Unterteils mit der Oberseite des Befestigungsmittels, sind grundsätzlich alle dem Fachmann bekannte Mittel verwendbar. So kann das Unterteil etwa durch Kleben, Verschrauben oder eine Schnappverbindung mit dem Befestigungsmittel drehfest verbunden werden. Bevorzugt ist es jedoch, zwei Schlitze an der Unterseite des Unterteils vorzusehen, in die entnehmbar ein bandförmiges Befestigungsmittel eingeführt ist. Ferner ist es bevorzugt, zwei derartige Befestigungsmittel parallel beabstandet in entsprechender Weise vorzusehen.

Die drehbare Verbindung des Unterteils mit dem Oberteil des Verbindungsmittels unter Beibehaltung einer im Wesentlichen planparallelen Zuordnung bei einem definierten Drehwinkel wird bevorzugt mechanisch, mittels einer Renk- oder einer Schraubverbindung, realisiert. Eine definierte Winkelbeziehung zwischen Unter- und Oberteil wird bei der Renkverbindung durch entsprechend angeordnete Stift-Schlitzpaarungen verwirklicht. Im Falle der alternativen Verwendung einer Schraubverbindung wird eine solche Winkelbeziehung durch Kontermittel bewirkt.

Eine Alternative zur mechanischen Verbindung stellt die gleichfalls bevorzugte magnetische Verbindung von Unterteil und Oberteil dar. Hierbei umfasst das Unterteil des Verbindungsmittels ein weiteres Mittel zum Erzeugen eines magnetischen Feldes, welches das Oberteil aus wenigstens teilweise magnetischem Material anzieht und auf diese Weise die beiden Teile verbindet. Zur Festlegung der gemeinsamen Drehachse des Unter- und Oberteils weisen die sich gegenüberliegenden Scheibenbereiche ein Erhebung und eine damit korrespondierende Vertiefung auf, die beide im Gebrauchszustand des Halters im Eingriff miteinander stehen.

Entsprechend der geometrischen Gestaltung der auf diese Weise ineinander greifenden Oberflächenbereiche ist per se jede gewünschte Winkelzuordnung zwischen Unter- und Oberteil möglich. Die Vertiefung kann dabei im Unterteil und die Erhebung im Oberteil vorgesehen sein oder umgekehrt. Bevorzugt ist es allerdings, die Vertiefung im Unterteil und die Erhebung im Oberteil vorzusehen, da u. a. das Oberteil dadurch mechanisch stabiler ausgeführt werden kann. Für eine kontinuierlich drehbare Zuordnung von Ober- und Unterteil sind die Erhebung und die Vertiefung zylinderförmig ausgebildet; für ein schrittweises relatives Drehen von Unter- und Oberteil ist die Ausbildung der Verbindungsbereiche in Form einer ineinander greifenden Außen- und Innenverzahnung vorgesehen, wobei die Positionsänderung durch Trennen von Unterteil und Oberteil und Wiedereinsetzen in der neuen gewünschten Winkelposition, also durch Umsetzen, erfolgt.

Die Zahl der möglichen Winkelpositionen des Haltemittels relativ zum Befestigungsmittel, bzw. des Oberteils des Verbindungsmittels relativ zu dessen Unterteil, ist auf diese Weise beliebig wählbar, da keinerlei Beschränkung hinsichtlich der Ausgestaltung der geometrischen Paarung besteht.

Darüber hinaus besteht die Möglichkeit, auch bei der kreisförmigen Ausführung der Verbindungsbereiche ein stufenweises Positionieren vorzusehen, indem die einander gegenüberliegenden Oberflächen mit entsprechenden Magnetpaarungen, vorzugsweise im Umfangsbereich, ausgebildet werden.

Als Befestigungsmittel zur lösbaren und sicheren Befestigung des Haltemittels auf der dorsalen Seite des nichtdominanten Unterarms des Operateurs ist wenigstens ein Armband vorgesehen, das jeweils bevorzugt als geschlossenes elastisches Band oder als offenes Band aus einem elastischen oder unelastischen Material, mit einem Verschluss ausgebildet ist und das jeweils aus sterilisierbarem, vorzugsweise wiederverwendbarem Material besteht. Alternativ kann wenigstens ein Gelenkband oder wenigstens ein elastisches Band aus Wegwerfmaterial vorgesehen sein.

Durch eine breitflächige Ausführung wird im Falle der Verwendung nur eines Befestigungsmittels das drehfeste Zusammenwirken mit dem Verbindungsmittel sichergestellt, wozu dieses, wie bereits angegeben, vorzugsweise durch zwei parallele Führungsschlitze, zur Aufnahme des bandförmigen Befestigungsmittels beiträgt und somit zuverlässig eine Relativdrehung zwischen Verbindungsmittel und Befestigungsmittel, und damit ein unerwünschtes Drehen des Haltemittels bzw. des Tabletts mit den darauf abgelegten Instrumenten während einer Operation, verhindert.

Derselbe Effekt ist mit zwei parallel beabstandeten und in gleicher Weise ausgebildeten Befestigungsmitteln zu erzielen. Durch die zwei Befestigungsmittel wird zudem die Sicherheit gegen ein unbeabsichtigtes Lösen während des bestimmungsgemäßen Gebrauchs des Halters erhöht sowie die Möglichkeit eröffnet, auch schmale band-, riemen- oder schnurförmige Befestigungsmittel einzusetzen.

Als Mittel zum Erzeugen des magnetischen Feldes auf der Oberfläche des Haltemittels, wie auch zum magnetischen Verbinden des Unterteils und Oberteils des Verbindungsmittels, gemäß der alternativ angegebenen Ausführungsform, werden bevorzugt Permanentmagnete in Kunststoff gekapselt eingesetzt. Form und Feldstärke der verwendeten Magnete werden dabei nach Form und Gewicht der zu halternden Teile, einschließlich der Instrumente, festgelegt und werden in bekannter Weise so bemessen, dass ein gleichermaßen sicheres Fixieren wie leichtes Trennen gewährleistet ist. Für unterschiedlich schwere Instrumente sind Haltemittel bzw. Tabletts mit entsprechend unterschiedlicher magnetischer Feldstärke und gegebenenfalls Feldverteilung vorgesehen, die durch eine geeignete Kodierung, etwa durch verschiedene Farbgebung, zuordenbar sind. Hinsichtlich der maximalen Betriebstemperatur der eingesetzten Permanentmagnete sind die aus der besonderen Verwendung des erfindungsgemäßen Halters resultierenden nachfolgend genannten Einschränkungen einzuhalten.

Sämtliche Einzelteile des erfindungsgemäßen Halters sind aus sterilen und/oder sterilisierbaren Materialien gefertigt. Der erfindungsgemäße Halter umfasst mithin ausschließlich solche Komponenten oder Materialien, die entweder bereits in steriler Form zur unmittelbaren Verwendung in einer sterilen Umgebung bereitgestellt werden, wie etwa die als Befestigungsmittel alternativ verwendeten Bänder aus Wegwerfmaterialien, oder die mit den für medizinische Gerätschaften in Labors und Kliniken üblicherweise angewandten Sterilisationsverfahren kompatibel sind. Zu diesen Sterilisationsverfahren zählen insbesondere die Dampfsterilisation und die Plasmasterilisation. Insgesamt müssen sämtliche Komponenten und Materialien des erfindungsgemäßen Halters somit den besonderen chemischen und physikalischen Anforderungen genügen, einschließlich den erhöhten Anforderungen an Temperaturstabilität und Desinfektionsmittelresistenz.

Entsprechend müssen die als Mittel zum Erzeugen eines magnetischen Feldes verwendeten Permanentmagnete eine maximale Betriebstemperatur aufweisen, die nicht kleiner als die während der Sterilisation des erfindungsgemäßen Halters auftretende Maximaltemperatur ist. Als maximale Betriebstemperatur wird die Temperatur bezeichnet, der ein Permanentmagnet ausgesetzt werden darf, ohne dass er seine Permanentmagneteigenschaften verliert. Sie liegt zumeist deutlich unterhalb der Curie-Temperatur des jeweiligen Permanentmagnetmaterials.

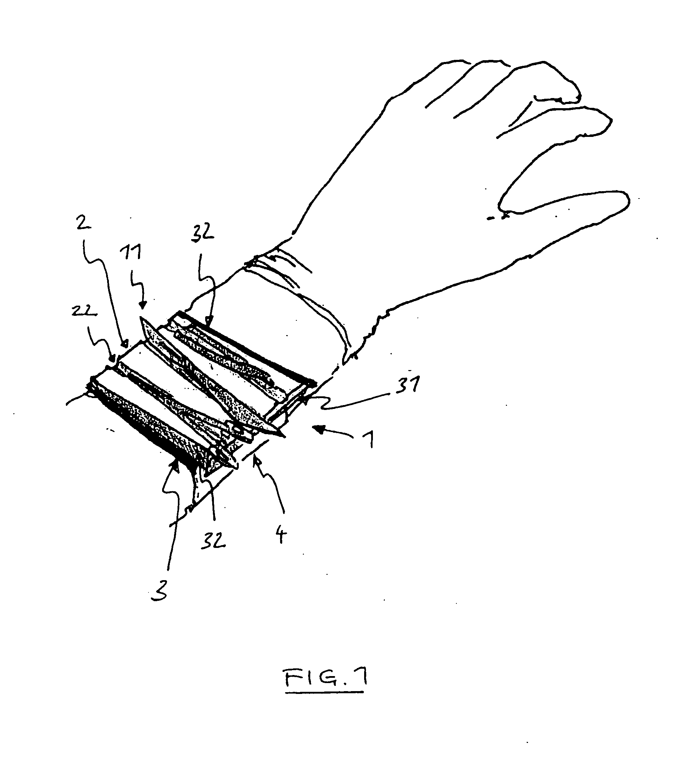

Einzelheiten der in den abhängigen Ansprüchen angegebenen vorteilhaften Ausführungsformen der Erfindung gehen auch aus den beiliegenden Zeichnungen hervor, auf die jeweils Bezug genommen wird, wobei
- Fig. 1: eine perspektivische Ansicht einer Ausführungsform des erfindungsgemäßen Halters im Gebrauch, mit Befestigungsmittel, einer Aufnahme mit Seitenwänden und einem darin geführten rechteckigen Haltemittel mit Rillen in der Oberseite und darin gehalterten Operationsinstrumenten (Pinzette, Skalpell, Nadelhalter) zeigt,

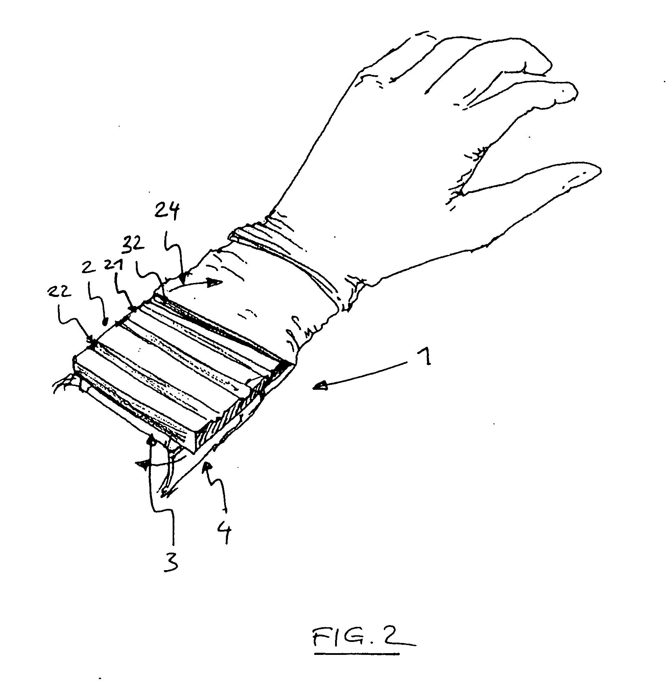

- Fig. 2: den erfindungsgemäßen Halter in Fig. 1 ohne Operationsinstrumente und unter Angabe der Drehrichtung des Haltemittels bezüglich des Befestigungsmittels zeigt,

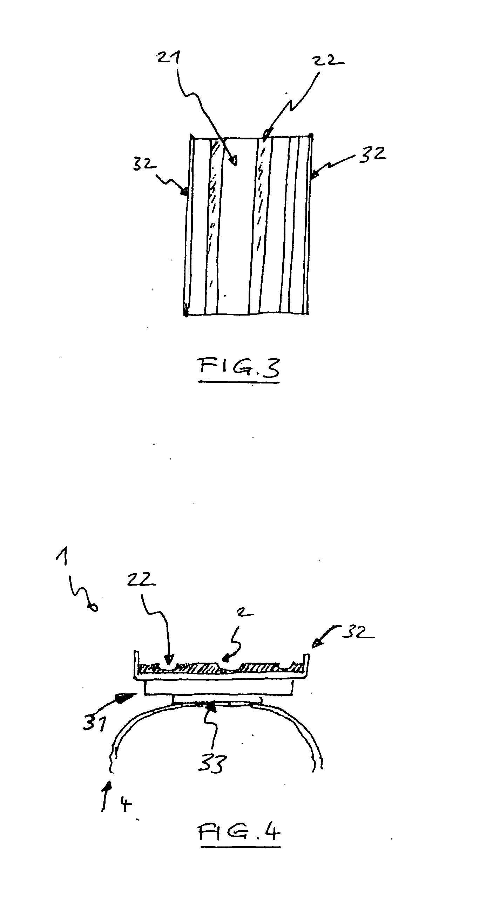

- Fig. 3: eine Draufsicht des erfindungsgemäßen Halters in Fig. 2 zeigt,
- Fig. 4: eine Vorderansicht des erfindungsgemäßen Halters in Fig. 2 zeigt,

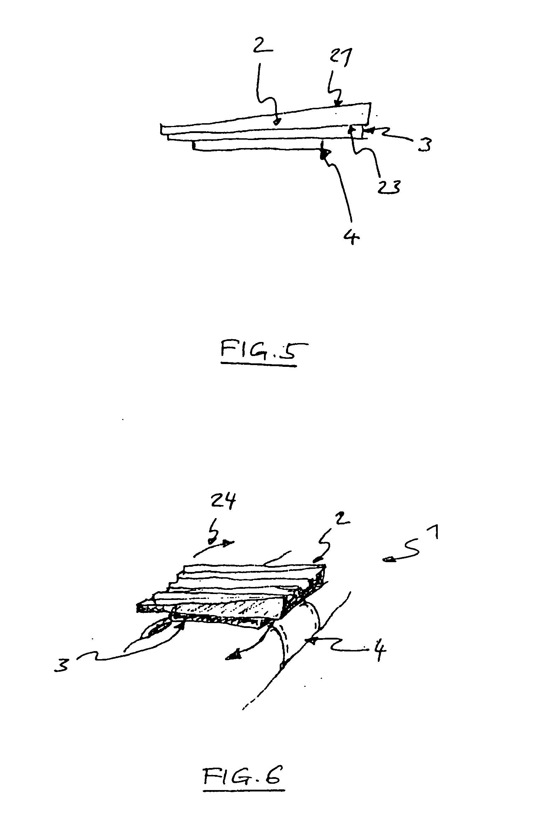

- Fig. 5: eine Seitenansicht einer weiteren Ausführungsform des erfindungsgemäßen Halters, mit Befestigungsmittel, Verbindungsmittel und einem Haltemittel mit einer gegenüber der Unterseite geneigten Oberseite zeigt,
- Fig. 6: den erfindungsgemäßen Halter in Fig. 5 in perspektivischer Ansicht im Gebrauch, unter Angabe der Drehrichtung des Haltemittels bezüglich des Befestigungsmittels zeigt,

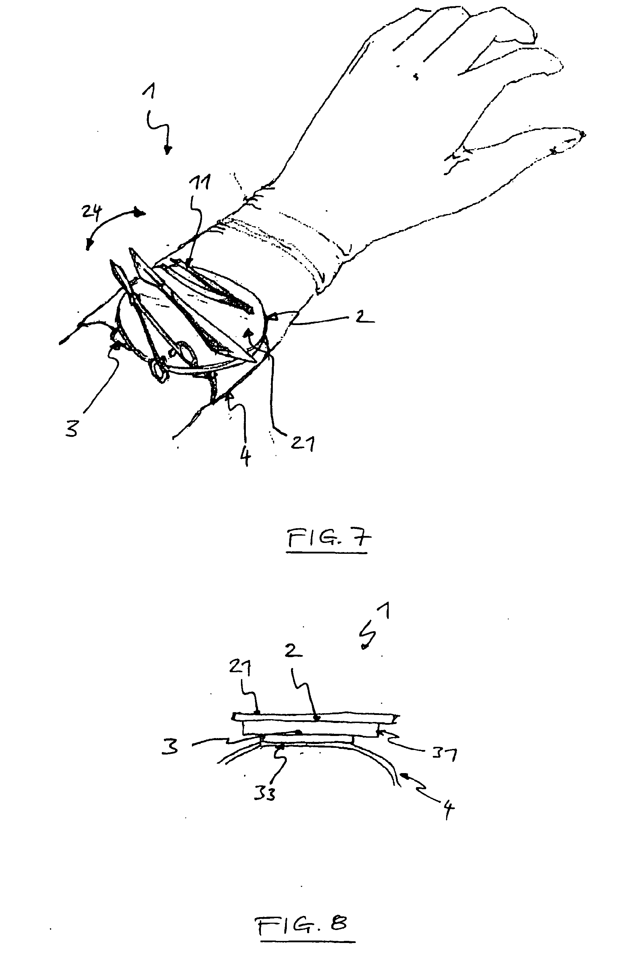

- Fig. 7: eine weitere Ausführungsform des erfindungsgemäßen. Halters in perspektivischer Ansicht im Gebrauch, mit einem runden Haltemittel mit ebener Oberseite und darauf gehalterten Operationsinstrumenten (Pinzette, Skalpell, Nadelhalter), unter Angabe der Drehrichtung des Haltemittels bezüglich des Befestigungsmittels, zeigt,
- Fig. 8: eine Vorderansicht des erfindungsgemäßen Halters in Fig. 7 zeigt,

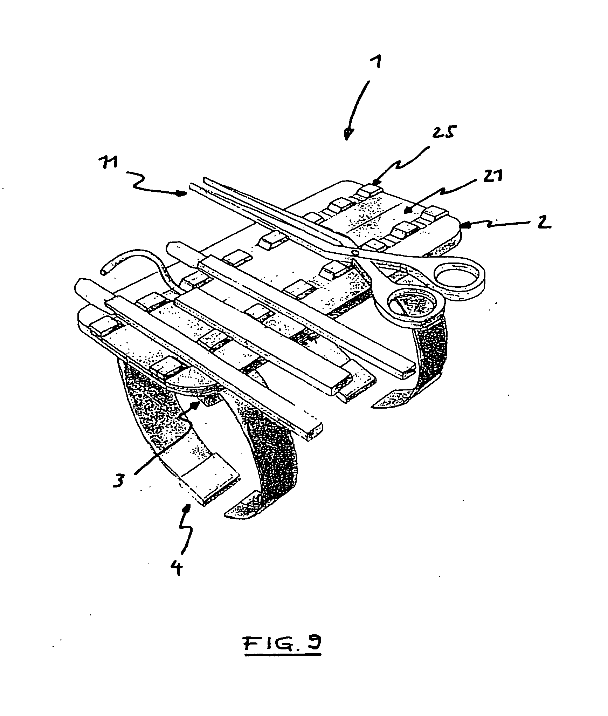

- Fig. 9: eine weitere Ausführungsform des erfindungsgemäßen Halters in perspektivischer Ansicht, mit einem rechteckigen Haltemittel mit Erhebungen auf der Oberseite und daran ausgerichteten Operationsinstrumenten (Nadelhalter, Skalpelle, Haken) sowie mit zwei Befestigungsmitteln zeigt, und wobei

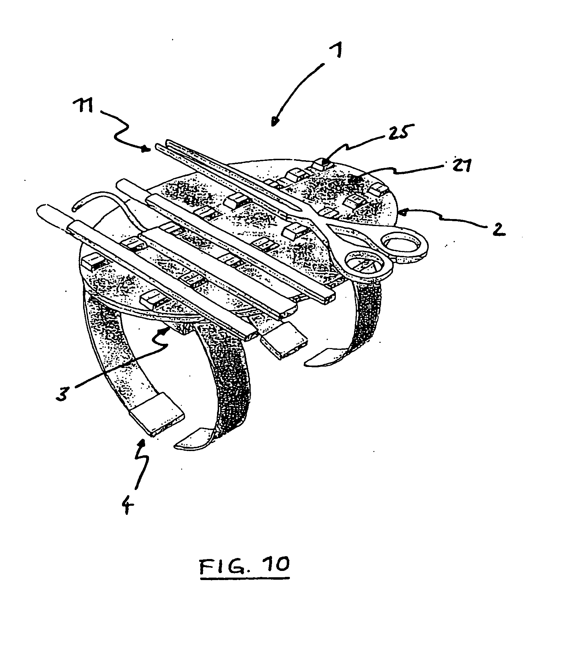

- Fig. 10: den erfindungsgemäßen Halter entsprechend Fig. 9, mit einem ovalen Haltemittel, anstelle des rechteckigen Haltemittels zeigt.

### Bezugszeichenliste

- 1: Halter
- 11: Operationsinstrumente
- 2: Haltemittel
- 21: Oberseite des Haltemittels
- 22: Rille in der Oberseite des Haltemittels
- 23: Unterseite des Haltemittels
- 24: Drehrichtung
- 25: Erhebung auf der Oberseite des Haltemittels
- 3: Verbindungsmittel
- 31: Oberteil des Verbindungsmittels / Aufnahme
- 32: Seitenwand der Aufnahme
- 33: Unterteil des Verbindungsmittels
- 4: Befestigungsmittel

## Patentansprüche

1. Halter (1) für Instrumente zur chirurgischen Behandlung des menschlichen oder tierischen Körpers, aufweisend ein Haltemittel (2) zum temporären Fixieren der Instrumente, ein Befestigungsmittel (4) zur lösbaren Befestigung des Haltemittels (2) am Arm eines Benutzers und ein Verbindungsmittel (3) zum Herstellen einer Verbindung zwischen dem Befestigungsmittel (4) und dem Haltemittel (2), wobei das Haltemittel (2) wenigstens ein Mittel zum Erzeugen eines magnetischen Feldes zum temporären Fixieren der Instrumente umfasst, das Haltemittel (2) schwenkbar oder drehbar mit einer festen Grundplatte verbunden ist, das Haltemittel (2) durch Arretierungsmittel in einer beliebigen Winkelposition relativ zur Grundplatte fixierbar ist, **dadurch gekennzeichnet, dass**
das Verbindungsmittel (3) im Wesentlichen zwei planparallele Scheiben umfasst, die um eine gemeinsame orthogonale Mittelachse drehbar gelagert und durch mechanische oder magnetische Mittel in ihrer planparallelen Zuordnung gehalten sind, wobei eine der Scheiben ein Unterteil (33) des Verbindungsmittels (3) bildet, die mit dem Befestigungsmittel (4) drehfest verbunden ist und die Grundplatte darstellt, und die andere Scheibe ein Oberteil (31) des Verbindungsmittels (3) bildet, das mit dem Haltemittel (2) verbunden ist.

2. Halter gemäß Anspruch 1, **dadurch gekennzeichnet, dass** das Haltemittel (2) eine im Wesentlichen ebene oder eine mit Rillen (22), Vertiefungen und/oder Erhebungen (25) versehene Oberseite (21) zum temporären Fixieren der Instrumente aufweist.

3. Halter gemäß Anspruch 2, **dadurch gekennzeichnet, dass** die Oberseite (21) des Haltemittels (2) im Wesentlichen parallel zu einer Unterseite (23) oder unter einem Winkel zwischen 0 und 30 Grad, insbesondere zwischen 5 und 15 Grad, zu der Unterseite (23) ausgebildet ist.

4. Halter gemäß Anspruch 3, **dadurch gekennzeichnet, dass** die magnetische Kraftwirkung im Wesentlichen ganzflächig, punktrasterförmig oder in Streifen von im Wesentlichen paralleler Ausrichtung auf der Oberseite (21) und auf der Unterseite (23) des Haltemittels (2) wirksam ist, wobei zur Ausbildung der magnetischen Kraftwirkung ein einziges magnetisches Felderzeugungsmittel in spezifischer Ausgestaltung oder eine Mehrzahl von gleichartigen magnetischen Felderzeugungsmitteln vorgesehen sind.

5. Halter gemäß einem der Ansprüche 1 bis 4, **dadurch gekennzeichnet, dass** das Verbindungsmittel (3) als Renk- oder Schraubverbindung mit dem Unterteil (33) und dem damit durch drehenden Eingriff verbindbaren Oberteil (31) ausgebildet ist, wobei der drehende Eingriff in einer Weise vorgesehen ist, dass eine feste Verbindung bei einer vorgegebenen Winkelzuordnung zwischen Unterteil (33) und Oberteil (31) im Gebrauchszustand des Halters besteht.

6. Halter gemäß Anspruch 5, **dadurch gekennzeichnet, dass** das Unterteil (33) lösbar mit dem Befestigungsmittel (4) verbunden ist.

7. Halter gemäß Anspruch 5 oder 6, **dadurch gekennzeichnet, dass** das Oberteil (31) in Form einer im Wesentlichen planparallelen Scheibe mit magnetischen Eigenschaften zum lösbaren Verbinden mit dem Haltemittel (2) ausgebildet ist.

8. Halter gemäß Anspruch 5 oder 6, **dadurch gekennzeichnet, dass** das Oberteil (31) in Form einer Aufnahme mit magnetischen Eigenschaften zum lösbaren Verbinden mit dem Haltemittel (2) und mit wenigstens teilweise umlaufendem Rand (32) zur lateralen Begrenzung des Haltemittels (2) im Gebrauchszustand des Halters ausgebildet ist.

9. Halter gemäß einem der Ansprüche 1 bis 4, **dadurch gekennzeichnet, dass** das Verbindungsmittel (3) das Unterteil (33) mit einem weiteren magnetischen Felderzeugungsmittel und das dazu unter mechanischem Eingriff positionierbaren magnetischen Oberteil (31) zum Herstellen einer temporären magnetischen Verbindung umfasst,
das wobei das Unterteil (33) eine Vertiefung und das Oberteil (31) eine korrespondierende Erhebung aufweisen, zur magnetischen Verbindung in einer vorgegebenen Winkelzuordnung zwischen Unterteil (33) und Oberteil (31) im Gebrauchszustand des Halters.

10. Halter gemäß Anspruch 9, **dadurch gekennzeichnet, dass** das magnetische Oberteil (31) lösbar mit dem Haltemittel (2) im Gebrauchszustand des Halters verbunden ist.

11. Halter gemäß Anspruch 9 oder 10, **dadurch gekennzeichnet, dass** das magnetische Oberteil (31) in Form einer Aufnahme zum lösbaren Verbinden mit dem Haltemittel (2) und mit wenigstens teilweise umlaufendem Rand (32) zur lateralen Begrenzung des Haltemittels (2) im Gebrauchszustand des Halters ausgebildet ist.

12. Halter gemäß einem der Ansprüche 9 bis 11, **dadurch gekennzeichnet, dass** das Unterteil (33) lösbar mit dem Befestigungsmittel (4) verbunden ist.

13. Halter gemäß einem der Ansprüche 1 bis 12, **dadurch gekennzeichnet, dass** das Befestigungsmittel (4) wenigstens ein geschlossenes elastisches Band oder wenigstens ein offenes Band mit einem Verschluss zur sicheren Befestigung am Arm eines Benutzers umfasst und drehfest mit dem Verbindungsmittel (3) zusammenwirkt.

14. Halter gemäß einem der Ansprüche 1 bis 13, **dadurch gekennzeichnet, dass** als Mittel zum Erzeugen eines magnetischen Feldes Permanentmagneten vorgesehen sind.

15. Halter gemäß einem der Ansprüche 1 bis 14, **dadurch gekennzeichnet, dass** die umfassten Einzelteile aus sterilen und/oder sterilisierbaren Materialien gefertigt sind.

## Claims

1. Holder (1) for instruments for surgical treatment of the human or animal body, comprising a holding means (2) for temporarily fixing the instruments, a fastening means (4) for detachably fastening the holding means (2) to the arm of a user, and a coupling means (3) for making a connection between said fastening means (4) and said holding means (2), wherein the holding means (2) comprises at least one means for generating a magnetic field for temporarily fixing the instruments, the holding means (2) being rotatable or pivotable connected to a firm base, the holding means (2) is fixable in any angular position with respect to said base by locking means, **characterized in that** the coupling means (3) comprises substantially two plane-parallel plates which are mounted around a common orthogonally central axis and are retained in their plane-parallel coordination by mechanical or magnetic means, wherein one of the plates forms a bottom part (33) of the coupling means (3), which is torque proof connected with the fastening means (4) and represents the firm base, and the other plate forms an upper part (31) of the coupling means (3), which is connected with the holding means (2).

2. Holder according to claim 1, **characterized in that** the holding means (2) has a substantially flat upper surface (21) or one comprising grooves (22), indentations, and/or projections (25) for temporarily fixing the instruments.

3. Holder according to claim 2, **characterized in that** the upper surface (21) of the holding means (2) being substantially formed parallel to the underside (23) or with an angle between 0 and 30 degrees, particularly between 5 and 15 degrees, to the underside (23).

4. Holder according to claim 3, **characterized in that** the magnetic action of force is substantially effective over the whole, in the form of a dot matrix or in stripes of substantially parallel alignment on the upper surface (21) and on the underside (23) of said holding means (2), with a single magnetic field generating means of specific design or a plurality of magnetic field generating means of the same kind for creating the magnetic action of force being provided.

5. Holder according to any one of claims 1 to 4, **characterized in that** the coupling means (3) is formed as a bayonet mount or screw joint with a bottom part (33) and an upper part (31) joinable to it by rotational engagement, wherein the rotational engagement being designated in a manner that at a predetermined angular position a strong connection between the bottom part (33) and the upper part (31) exists in a condition for purpose of the holder.

6. Holder according to claim 5, **characterized in that** the bottom part (33) is detachably connected on the fastening means (4).

7. Holder according to claim 5 or 6, **characterized in that** the upper part (31) is formed in the form of a substantially plane-parallel plate with magnetic properties for detachably connecting with said holding means (2).

8. Holder according to claim 5 or 6, **characterized in that** the upper part (31) has the form of a receptacle with magnetic properties for detachably connecting with said holding means (2) and with an at least in part circumferentially extending border (32) for lateral restriction of said holding means (2) in a condition for purpose of the holder.

9. Holder according to any one of claims 1 to 4, **characterized in that** said coupling means (3) comprises the bottom part (33) with a further means for generating a magnetic field and the magnetic upper part (31) being positionable in mechanical engagement thereto for making a temporary magnetic coupling, wherein the bottom part (33) having a recession and the upper part (31) having a correspondent projection for a magnetic coupling in a predetermined angular position between bottom part (33) and upper part (31) in a condition for purpose of the holder.

10. Holder according to claim 9, **characterized in that** the magnetic upper part (31) is detachably connected with the holding means (2) in a condition for purpose of the holder.

11. Holder according to claim 9 or 10, **characterized in that** the magnetic upper part (31) has the form of a receptacle for detachably connecting with the holding means (2) and with an at least in part circumferentially extending border (32) for lateral restriction of said holding means (2) in a condition for purpose of the holder.

12. Holder according to claims 9 to 11, **characterized in that** the bottom part (33) is detachably connected on the fastening means (4).

13. Holder according to claims 1 to 12, **characterized in that** the fastening means (4) is at least one closed elastic ribbon or at least one open ribbon with a fastener for securely fastening on the arm of a user and is in torque proof cooperation with said coupling means (3).

14. Holder according to any one of claims 1 to 13, **characterized in that** permanent magnets are provided as means for generating a magnetic field.

15. Holder according to any one of claims 1 to 14, **characterized in that** the component parts comprised are made of sterile and/or sterilizable materials.

## Revendications

1. Support (1) pour des instruments de traitement chirurgical de corps humains ou animaux, comportant un moyen de maintien (2) pour la fixation temporaire des instruments, un moyen de fixation (4) pour la fixation amovible du moyen de maintien (2) au bras d'un utilisateur, et un moyen de connexion (3) pour réaliser une connexion entre le moyen de fixation (4) et le moyen de maintien (2), le moyen de maintien (2) comprenant au moins un moyen de génération d'un champ magnétique permettant la fixation temporaire des instruments, le moyen de maintien (2) étant connecté de manière pivotante ou rotative à une plaque de base fixe, le moyen de maintien (2) pouvant être fixé par des éléments d'arrêt dans une position angulaire quelconque par rapport à la plaque de base, **caractérisé en ce que** le moyen de connexion (3) comporte sensiblement deux disques dont les plans sont parallèles, lesquels sont montés rotativement autour d'un axe central orthogonal et maintenus dans leur agencement parallèle planaire par des moyens mécaniques ou magnétiques, un des disques formant une pièce inférieure (33) du moyen de connexion (3), connectée solidairement en rotation au moyen de fixation (4) et constituant la plaque de base, et l'autre disque formant une pièce supérieure (31) du moyen de connexion (3), connectée au moyen de maintien (2).

2. Support selon la revendication 1, **caractérisé en ce que** le moyen de maintien (2) présente une face supérieure (21) sensiblement plane ou pourvue de gorges (22), d'évidements et/ou de saillies (25) pour la fixation temporaire des instruments.

3. Support selon la revendication 2, **caractérisé en ce que** la face supérieure (21) du moyen de maintien (2) est prévue sensiblement parallèle à une face inférieure (23) ou forme avec ladite face inférieure (23) un angle compris entre 0 et 30 degrés, en particulier entre 5 et 15 degrés.

4. Support selon la revendication 3, **caractérisé en ce que** la force magnétique est efficace sensiblement sur toute la surface, en formant une trame de points ou des bandes sensiblement parallèles sur la face supérieure (21) et sur la face inférieure (23) du moyen de maintien (2), un seul moyen de génération de champ magnétique, de configuration spécifique, ou une pluralité de moyens de génération de champ magnétique similaires étant prévus pour la formation de la force magnétique.

5. Support selon l'une des revendications 1 à 4, **caractérisé en ce que** le moyen de connexion (3) est réalisé comme connexion à baïonnette ou à vis avec une pièce inférieure (33) et une pièce supérieure (31) pouvant lui être connectées par enclenchement rotatif, ledit enclenchement rotatif étant prévu de manière à créer une connexion fixe avec un agencement angulaire défini entre la pièce inférieure (33) et la pièce supérieure (31) en état d'utilisation du support.

6. Support selon la revendication 5, **caractérisé en ce que** la pièce inférieure (33) est connectée de manière amovible au moyen de fixation (4).

7. Support selon la revendication 5 ou 6, **caractérisé en ce que** la pièce supérieure (31) est réalisée sous la forme d'un disque de plan sensiblement parallèle avec des propriétés magnétiques pour la connexion amovible au moyen de maintien (2).

8. Support selon la revendication 5 ou 6, **caractérisé en ce que** la pièce supérieure (31) est réalisée sous la forme d'une réception avec des propriétés magnétiques pour la connexion amovible au moyen de maintien (2), et avec un bord (32) au moins partiellement périphérique pour la délimitation latérale du moyen de maintien (2) en état d'utilisation du support.

9. Support selon l'une des revendications 1 à 4, **caractérisé en ce que** le moyen de connexion (3) comprend la pièce inférieure (33) avec un autre moyen de génération d'un champ magnétique et la pièce supérieure (31) magnétique positionnable sur celle-ci par enclenchement mécanique pour la réalisation d'une connexion magnétique temporaire, la pièce inférieure (33) présentant un évidement et la pièce supérieure (31) une saillie correspondante pour la connexion magnétique suivant un agencement angulaire défini entre la pièce inférieure (33) et la pièce supérieure (31) en état d'utilisation du support.

10. Support selon la revendication 9, **caractérisé en ce que** la pièce supérieure (31) magnétique est connectée de manière amovible au moyen de maintien (2) en état d'utilisation du support.

11. Support selon la revendication 9 ou 10, **caractérisé en ce que** la pièce supérieure (31) magnétique est réalisée sous la forme d'une réception pour la connexion amovible au moyen de maintien (2), et avec un bord (32) au moins partiellement périphérique pour la délimitation latérale du moyen de maintien (2) en état d'utilisation du support.

12. Support selon l'une des revendications 9 à 11, **caractérisé en ce que** la pièce inférieure (33) est connectée de manière amovible au moyen de fixation (4).

13. Support selon l'une des revendications 1 à 12, **caractérisé en ce que** le moyen de fixation (4) comprend au moins une bande élastique close ou au moins une bande ouverte avec une fermeture pour une fixation sûre au bras d'un utilisateur, et **en ce qu'**il coopère solidairement en rotation avec le moyen de connexion (3).

14. Support selon l'une des revendications 1 à 13, **caractérisé en ce que** des aimants permanents sont prévus en tant que moyens de génération d'un champ magnétique.

15. Support selon l'une des revendications 1 à 14, **caractérisé en ce que** les pièces détachées incluses sont fabriquées avec des matériaux stériles et/ou stérilisables.
